# EUROPEAN PATENT APPLICATION

(11) **EP 3 053 618 A1**
(43) Date of publication of application: **10.08.2016**
(21) Application number: 15382040.2
(22) Date of filing: 06.02.2015
(51) Int. Cl.: A61M 5/142

(54) **Infusion pump for enteral nutrition**

(71) Applicant: Fernandez Morera, Juan Luis, 33006 Oviedo (Asturias) (ES)
(72) Inventor: Fernandez Morera, Juan Luis, 33006 Oviedo (Asturias) (ES)
(74) Representative: Carvajal y Urquijo, Isabel

(57) **Abstract**

Infusion pump for enteral nutrition, which comprises a case (2) wherein a manually operated peristaltic pumping mechanism (10) is housed, which is designed to enable a liquid solution to flow from a bottle (B) through a flexible feeding tube (5), between an inlet end (4) and an outlet end (6) thereof. Said pump (1), comprises a housing (20) designed to house the bottle (B), which presents an upper opening (21) that enables the introduction of the bottle (B) and a bottom base (22); and a needle (3) arranged in the bottom base (22) with an upper end (31) designed to puncture the bottle (B) and a lower end (32) that is connected to the inlet end (4), said needle (3) having a passage (33) intended to communicate the solution contained in the bottle (B) and the feeding tube (5).

## Description

### Technical field

The present invention relates to an infusion pump for enteral nutrition, designed to administer enterally (via a nasogastric, gastrostomy or jejunostomy probe) liquid infusions (such as nutritional formulas, drugs or water). The device of the present invention is particularly indicated for use in hospitals or at home, promoting both patients' self-care and autonomy, whilst facilitating the caregivers' activity.

### Background of the invention

Enteral nutrition is a special nutritional technique, which belongs to the artificial nutrition field, consisting in the administration of liquid solutions through the use of tubes. These tubes comprise a proximal end outside the patient's body and a distal end located in their digestive system (stomach, duodenum or jejunum). The proximal end is accessible for its handling, so as to enable the introduction of these nutritional elements into the digestive apparatus of the patients. This technique is particularly indicated for those individuals that experiment difficulties to carry out an appropriate active oral intake, provided that their digestive system is able to absorb nutrients.

The administration of these liquid solutions via tubes can be done continuously, by gravity or using automatic perfusion pumps; or it can be done using an intermittent feeding pattern through the instillation by means of large-volume syringes (that may contain 100 cc). This latter method is the most used for patients treated as outpatients where the pattern of use simulates the usual human intake schedules.

The use of these syringes in the intermittent enteral nutrition entails significant risks, mainly related with an incorrect administration of the nutritional formula. Abdominal pain, vomiting, nausea and gastroesophageal or ostomy reflux, represent most of the complications associated with this type of administration. Their origin lies in the technical difficulties typically encountered by both caregivers and patients themselves in order to deliver a constant volume supply of the nutritional formula in a given time period (trying not exceed 10 cc per minute). This entails a great dedication from caregivers and a significant loss of autonomy for patients, worsened even more, when taking into account that many of these patients usually suffer from other associated diseases that reduce their motor ability, such as Parkinson's disease, Alzheimer, miastenia gravis, Guillain-Barré syndrome, amyotrophic lateral sclerosis - or sequels after a stroke. In addition, another serious technical problem to take into account is related with difficulties in handling the nutritional formula, whose dosage has to be extracted from larger volume containers.

Moreover, automatic perfusion pumps are mainly used to deliver drugs via intravenous perfusion, parenteral nutrition, or continuous enteral nutrition (from 12 to 24 hours of continuous perfusion). They are rarely used in intermittent enteral feeding patterns or in outpatients.

In the field of parenteral nutrition, it is known the use of electronic peristaltic pumps. For example, like those disclosed in documents EP0447909A1 and EP0521184A1 for a continuous intravenous administration. This type of pumps has significant disadvantages, mainly associated to the high complexity they involve. Namely, the use of flow and pressure sensors requires qualified personnel to operate and program them, in addition to a proper and meticulous maintenance. Moreover, they depend from power sources to operate. They are cumbersome and heavy, which makes it considerably difficult to transport and handle them. All of this has a remarkable negative effect on the autonomy, for both patients and caregivers. Finally, their high cost reduces even more their use, which is substantially limited to hospital level.

Furthermore, within the parenteral nutrition context, and in applications more specific, it is known to use some devices that partially solve these problems, making these devices independent from a power source. For example, document US2012/022456A1 discloses a manually operable peristaltic pump for intravenous infusion. This pump comprises a piezoelectric system associated with the movement of the manual operation that is designed to regulate the speed of the pump rotor. In this sense, the pump presents a complex operation and transmission mechanism, in addition to the piezoelectric system itself, which involves a large number of parts, a higher risk of malfunction, more complex repairs, low adaptability to eventual changes in the design of the device in order to facilitate its use, etc. At the same time, the device is specifically designed to work with bags of fluids (such as physiological saline, Ringer's Lactate, etc.) external to device, and a fluid line that is connected to the device, hangs freely from each one of them. These bags require the use of stands or poles, which together with the hanging fluid lines, hamper considerably the patient's mobility and entail a greater loss of autonomy.

Now, in the field of intermittent enteral nutrition, document EP2253345A1 shows a peristaltic pump for the infusion of semi-solid nutritional supplements (high density and/or viscosity foods). This pump incorporates a manually operated peristaltic pumping mechanism, designed to enable the semi-solid nutritional supplement to circulate through a flexible feeding tube, between one inlet end of said feeding tube, which receives the nutritional supplement and an outlet end thereof, which is connected to the proximal end of the administrating tube, said circulation being originated by the compression of the feeding tube due to the motion of a rotor with perimeter rollers. As in the previous case, this device is specifically designed to work with bags external to the device, and a feeding line that is connected to the device, hangs freely from each one of them. In this case, this aspect is even more relevant, given that the semi-solid nutritional supplements have a significant viscosity, the furthest the difference in height between the bag and the device is, more the circulation of the supplement through the feeding tube is favoured. Therefore, as it has been previously mentioned, these bags require the use of stands or poles that together with the hanging feeding lines, hamper considerably the patient's mobility and entail a greater loss of autonomy. Finally, the design of this pump also presents a little or no adaptability to eventual changes in the device design to facilitate its use.

The present invention solves the aforementioned drawbacks, by means of a infusion pump for intermittent enteral nutrition, which is highly functional, compact and simple, designed to administer enterally (via a nasogastric, gastrostomy or jejunostomy tube) liquid solutions (such as nutritional formulas, drugs or water) in a convenient and secure way to the patients. The present invention enables the integration of the bottle or vessel containing the liquid solution in the device itself, thus facilitating it transportation and promoting the patient's mobility. Furthermore, its design is simple, its adaptation to different use conditions and its easy handling, promote both patients' self-care and autonomy, whilst facilitating the caregivers' activity.

### Description of the invention

The infusion pump for enteral nutrition of the present invention, comprises a case wherein a manually operated peristaltic pumping mechanism is housed, designed to enable a liquid solution contained in a bottle to flow through a flexible feeding tube, between an inlet end of said feeding tube, which receives the solution from the bottle and an outlet end thereof. Wherein said outlet end is designed to be connected to the proximal end of an administration tube present in the patient's body.

Said pump comprises:
- a housing or receptacle arranged in the case designed to house the bottle, which in turn presents an upper opening that enables the introduction of the bottle and a bottom base; and
- a needle arranged in the bottom base, which in turn has an upper end designed to puncture the bottle and a lower end that is connected to the inlet end, said needle having a longitudinal communication passage designed to establish a fluid communication between the solution contained in the bottle and the feeding tube.

Therefore, as part of this operative compact structure, the inlet end is directly connected to the bottle that contains the nutritional formula, which reduces considerably the handling of the bottle. This optimises the organoleptic characteristics, since as there is less contact with the external environment both oxidation and the risk of said formula being contaminated are reduced.

The pump of the present invention is compatible with any bottle or vessel, whose shape and size fits into the housing of the case, and that in addition it can be perforated by the needle to extract its contents, either by puncturing directly the body of the bottle itself, or a plug or any other closing element and/or seal thereof. In any case, the puncture must be safe and at the same time it must avoid any eventual leakage of the liquid solution once the bottle has been perforated. To this end, preferably the bottle is provided with a disposable threaded plug, with a central part made of latex, so that said bottle can be housed in a reversed position into the housing, i.e., with the plug down, so the needle punctures the latex seal of the plug.

Preferably, the case is formed by a first side and by a second side than can be easily coupled, for example by means of screws and/or snap-fit elements and/or clipping (tabs, shoulders, recesses, etc.), to facilitate the pump assembly, as well as eventual repair works thereon.

Preferably, the pumping mechanism comprises a drive shaft designed to rotate under the action of a crank, where said crank can be easily operated manually both by the patients themselves or their caregiver. Said drive shaft comprises in turn a first end arranged on a first side of the case, and a second end arranged on a second side of the case opposite to the first side; whereas the crank comprises a connecting element to be joined to the drive shaft designed to be interchangeable assembled either on the first end or on the second end. To provide stability to the junction between the crank and the drive shaft, and at the same time facilitate their exchangeability by the user (patient or caregiver), it is possible to use permanent magnets or fast-coupling mechanical means (for instance hooks, or clipping elements etc.) between the crank and the drive shaft. This way, the pump can also be easily adapted to its use for right-handed and left-handed people, as well as to the handling of the device by the patient, or the caregiver that will be presented with said device.

Preferably, the first end is supported by a first bearing integral to the case, which in turn enables the rotation of said first end; whereas the second end is supported by a second bearing integral to the case, which in turn enables the rotation of said second end.

Preferably, the pumping mechanism comprises a transmission shaft arranged in parallel relative to the drive shaft; and a transmission belt designed to transmit the rotation from the drive shaft to the transmission shaft.

Preferably, the pumping mechanism comprises a rotor provided with a plurality of perimeter rollers designed to press the feeding tube, causing the content of the bottle to flow through said tube; and a cylindrical chamber into which the rotor is coaxially housed, so the feeding tube is arranged between the cylindrical chamber and the rollers. Moreover, the cylindrical chamber comprises an inlet opening with an inlet deflector that redirects the feeding tube towards the housing, and an outlet opening with an outlet deflector that redirects the feeding tube towards an outlet hole of the case.

Preferably, the drive shaft comprises a toothed perimeter section that meshes with the transmission belt; whereas the transmission shaft comprises a cogwheel that meshes with the transmission belt, and a transmission end, integral with the rotor.

Preferably, the housing comprises a slit extending downwards from the upper opening, until the proximity of the bottom base. This slit, enables the user to see at all moments the content level of the bottle without having to remove it previously.

In order to facilitate the maintenance works of the pump, preferably the case comprises a maintenance opening that provides access to the peristaltic mechanism and to the needle, and which in turn is covered with a detachable plate. The case in turn comprises a closing tab designed to slide over the detachable plate and enable the attachment thereof on the maintenance opening. This opening enables to repair and replace the needle and the feeding tube.

In order to facilitate the pump operation, preferably the case comprises a handle designed to be assembled, in an interchangeable manner, on first hooking means arranged on a first side of the case, and on second hooking means arranged on a second side of the case, opposite to the first side. In this way, the pump can be easily adapted to its use for right-handed and left-handed people, by having the crank arranged on one side and the handle on the opposite side.

In order to keep the outlet end in a tidy arrangement when the pump is not in use, preferably the case comprises a plurality of fastening clamps, designed to fasten an outer length of the feeding tube.

Preferably, the pump comprises resilient locking means designed to hold the bottle in position once it has been arranged into the housing.

As part of the security and servicing processes of the device of the present invention, within the regulatory framework on enteral nutrition, both the needle and the feeding tube through which the liquid runs are replaceable. To this end, preferably the needle comprises a threaded passage designed to be screwed onto the bottom base of the housing, and at the same time to be able to be unscrewed from said base so as to be extracted, together with the tube in its entirety. The tube and the needle are joined and sealed, so both elements are extracted at the same time.

According to the described design, the pump of the present invention presents with respect to known techniques, the following advantages:
- It simplifies the administration techniques within the field of intermittent enteral nutrition, facilitating the task for caregivers while promoting the self-care and autonomy of the patients.
- By means of the controlled administration of a volume of the nutritional formula per unit of time the risks of associated complications are reduced, such as abdominal pain, nauseas, dumping, gastroesophageal reflux, bronchoaspiration and diarrhoea.
- It promotes the self-care processes and functional autonomy of the patient, with a subjective health improvement perceived and a satisfactory nutritional state of the same.
- It improves the relationship between the patient and the caregiver, optimises the external supplementary sanitary assistance time, and improves the assistance quality as well as the working context of the caregiver.
- Decreases the manipulation requirements of the nutritional formula, given that it is a closed system and increases the patient's security.

### Brief description of the drawings

What follows is a very brief description of a series of drawings that facilitate a better understanding of the invention, and which are expressly related to a preferred embodiment of said invention, presented by way of non-limiting examples thereof.
Figure 1 represents a perspective view of the pump of the present invention.
Figure 2 represents an internal perspective view of the pump, wherein the first side of the case has been removed.
Figure 3 represents an internal perspective view of the pump, wherein the second side of the case has been removed.
Figure 4 represents a detailed view of figure 3.
Figure 5 represents a perspective view of the peristaltic pumping mechanism.
Figure 6 represents a perspective view of the pump showing the maintenance opening.
Figure 7 represents a perspective view of the pump showing the handle.
Figure 8 represents an exploded perspective view of the pump.

### Detailed description of the invention

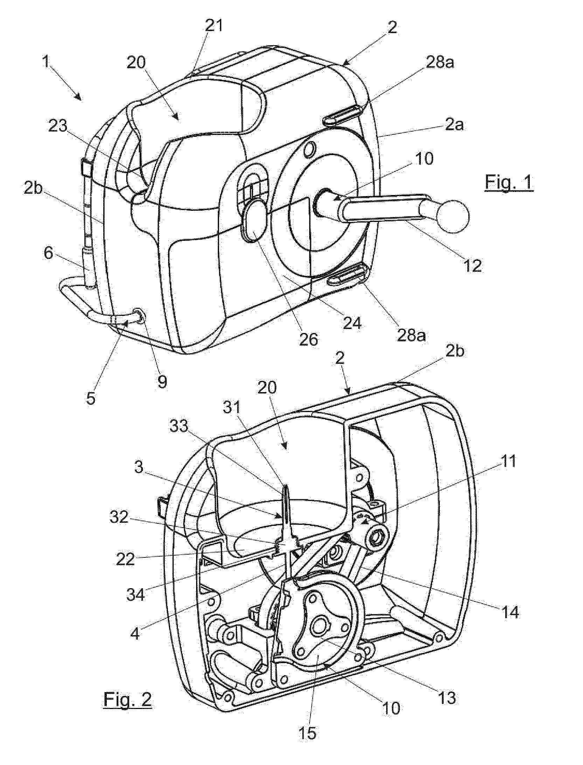

As it can be appreciated in figures 1-3, the infusion pump (1) for enteral nutrition of the present invention, comprises a case (2) wherein a manually operated peristaltic pumping mechanism (10) is housed, designed to enable a liquid solution contained in a bottle (B) to flow through a flexible feeding tube (5), between an inlet end (4) of said feeding tube (5) that receives the solution from the bottle (B) and an outlet end (6) thereof. Wherein said outlet end (6) is designed to be connected to the proximal end of an administration tube.

Said pump (1) comprises:
- a housing (20) arranged in the case (2) designed to house the bottle (B), which in turn presents an upper opening (21) that enables the introduction of the bottle (B) and a bottom base (22); and
- a needle (3) arranged in the bottom base (22) which in turn has an upper end (31) designed to puncture the bottle (B) and a lower end (32) that is connected to the inlet end (4), said needle (3) having a longitudinal communication passage (33) designed to establish a fluid communication between the solution contained in the bottle (B) and the feeding tube (5).

The housing (20) comprises a slit (23) extending downwards from the upper opening (21), until the proximity of the bottom base (22), through which the user can see at all moments the content level of the bottle (B) without having to remove it previously.

As it can be appreciated, the case (2) is formed by a first side (2a) and by a second side (2b) than can be easily coupled, to facilitate the pump assembly, as well as eventual repair works thereon.

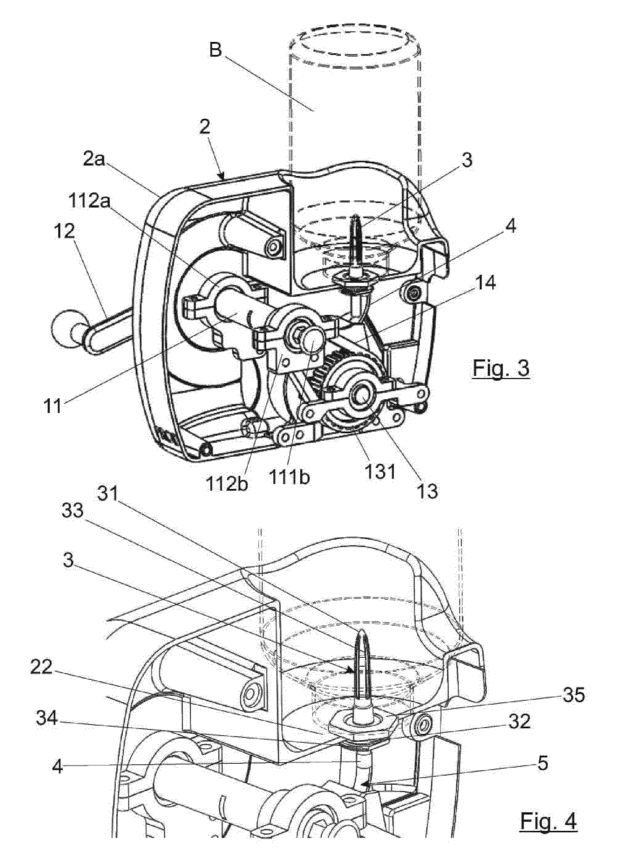

As it can be appreciated in figure 4, the needle (3) comprises a threaded passage (34) designed to be screwed onto the bottom base (22) of the housing (20), in this case through a nut (35) arranged on said bottom base (22), and at the same time to be able to be unscrewed therefrom so as to be extracted. Both the needle (3) and the feeding tube (5) are replaceable.

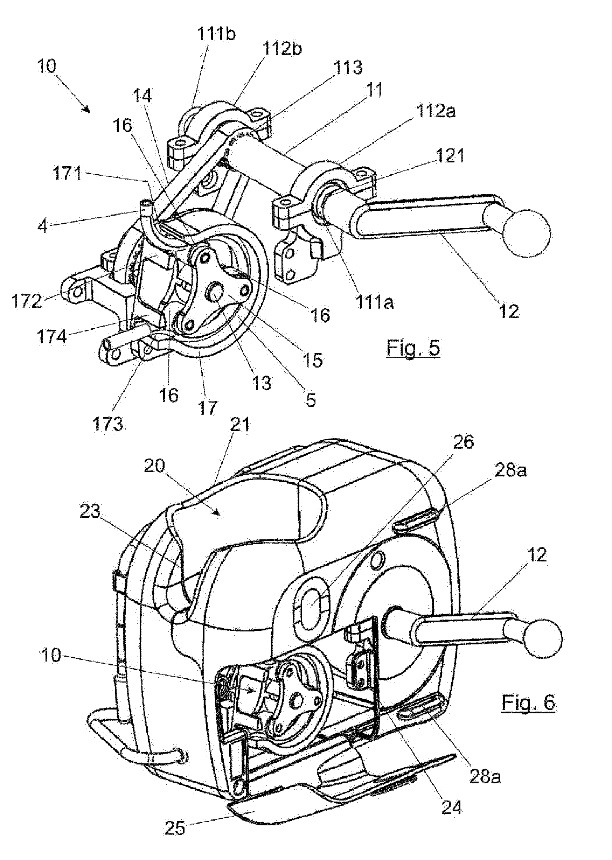

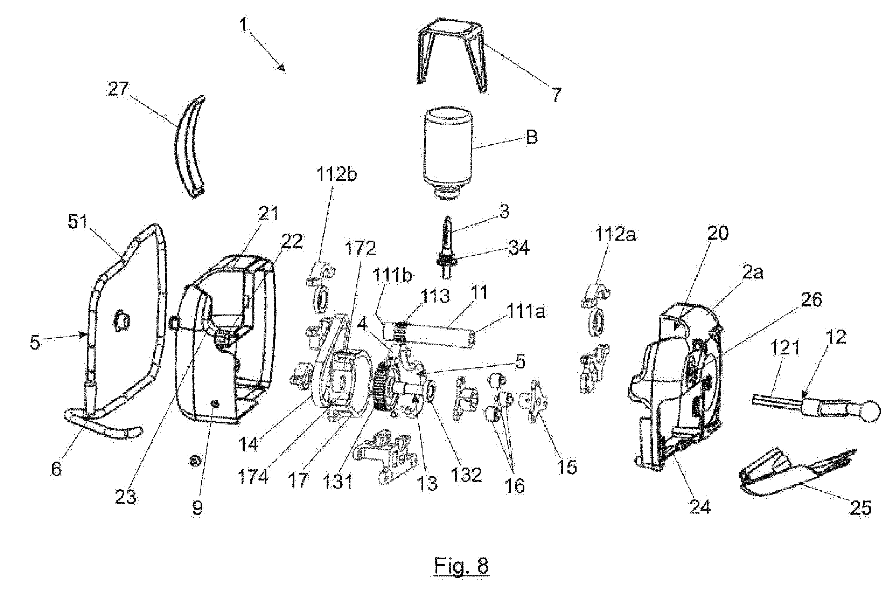

As it can be appreciated in figure 5, the pumping mechanism (10) comprises a drive shaft (11) designed to rotate under the action of a crank (12). Said drive shaft (11) comprises in turn a first end (111a) arranged on a first side (2a) of the case (2), and a second end (111b) arranged on a second side (2b) of the case (2) opposite to the first side (2a). The crank (12) comprises a connecting element (121) to the drive shaft (11) designed to be interchangeably assembled either on the first end (111a) or on the second end (111b). According to the present example, said connecting element (121), which is better appreciated in figure 8, corresponds to a rod having a polygonal cross-section coincident with the longitudinal socket of the drive shaft (11), designed to transmit the movement from the crank (12) to said drive shaft (11).

The first end (111a) is supported by a first bearing (112a) integral to the case (2), which in turn enables the rotation of said first end (111a); whereas the second end (111b) is supported by a second bearing (112b) integral to the case (2), which in turn enables the rotation of said second end (111b).

The pumping mechanism (10) comprises a transmission shaft (13) arranged in parallel relative to the drive shaft (11); and a transmission belt (14) designed to transmit the rotation of the drive shaft (11) to the transmission shaft (13).

The pumping mechanism (10) comprises a rotor (15) provided with a plurality of perimeter rollers (16) designed to press the feeding tube (5); and a cylindrical chamber (17) into which the rotor (15) is coaxially housed, so the feeding tube (5) is arranged between the cylindrical chamber (17) and the rollers (16). Moreover, the cylindrical chamber (17) comprises an inlet opening (171) with an inlet deflector (172) that redirects the feeding tube (5) towards the housing (20), and an output opening (173) with an outlet deflector (174), which redirects the feeding tube (5) towards an output hole (9) of the case (2), figure 1.

The drive shaft (11) comprises a toothed perimeter section (113) that meshes with the transmission belt (14); whereas the transmission shaft (13) comprises a cogwheel (131) that meshes with the transmission belt (14), and a transmission end (132), integral with the rotor (15). figure 8.

As it can be appreciated in figure 6, in order to facilitate the maintenance works, the case (2) comprises a maintenance opening (24) that provides access to the peristaltic mechanism (10) and the needle (3), which in turn is covered with a detachable plate (25). The case (2) in turn comprises a closing tab (26) designed to slide over the detachable plate (25) and enable the attachment thereof on the maintenance opening (24). This opening enables to repair and replace the needle and the feeding tube.

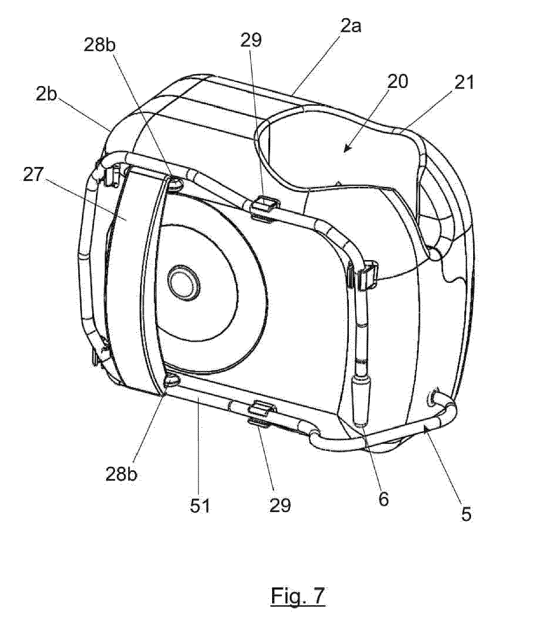

As it can be appreciated in figure 7, to facilitate the operation of the pump (1), the case (2) comprises a handle (27) designed to be assembled in an interchangeable manner, on first hooking means (28a) arranged on a first side (2a) of the case (2), figure 6, or on second hooking means (28b) arranged on a second side (2b) of the case (2), opposite to the first side (2a). In this way, the pump (1) can also be easily adapted to its use for right-handed and left-handed people, by arranging the crank (12) on one side (2a, 2b) and the handle (27) on the opposite side (2a, 2b).

In order to keep the outlet end (6) in a tidy arrangement when the pump (1) is not in use, the case (2) comprises a plurality of fastening clamps (29) designed to fasten an outer length (51) of the feeding tube (5).

Figure 8 shows an exploded perspective view of the pump (1) wherein it can be better appreciated the various components thereof. As it can be seen, the pump (1) comprises resilient locking means (7) designed to hold the bottle (B) in position once it has been arranged into the housing (20).

## Claims

1. An infusion pump for enteral nutrition, which comprises a case (2) wherein a manually operated peristaltic pumping mechanism (10) is housed, designed to enable a liquid solution contained in a bottle (B) to flow through a flexible feeding tube (5), between an inlet end (4) of said feeding tube (5), which receives the solution from the bottle (B) and an outlet end (6) thereof, said pump (1) being **characterised in that** it comprises:
- a housing (20) arranged in the case (2) designed to house the bottle (B), which in turn presents an upper opening (21) that enables the introduction of the bottle (B) and a bottom base (22); and
- a needle (3) arranged in the bottom base (22) which in turn has an upper end (31) designed to puncture the bottle (B) and a lower end (32) that is connected to the inlet end (4), said needle (3) having a longitudinal communication passage (33) designed to establish a fluid communication between the solution contained in the bottle (B) and the feeding tube (5).

2. The infusion pump for enteral nutrition according to claim 1 **characterised in that** the pumping mechanism (10) comprises a drive shaft (11) designed to rotate under the action of a crank (12).

3. An infusion pump for enteral nutrition according to claim 2 **characterised in that** the drive shaft (11) comprises a first end (111a) arranged on a first side (2a) of the case (2), and a second end (111b) arranged on a second side (2b) of the case (2) opposite to the first side (2a); **and in that** the crank (12) comprises a connecting element (121) to the drive shaft (11) designed to be interchangeably assembled either on the first end (111a) or on the second end (111b).

4. An infusion pump for enteral nutrition according to claim 3 **characterised in that** the first end (111a) is supported by a first bearing (112a) integral to the case (2), which in turn enables the rotation of said first end (111a); and **in that** the second end (111b) is supported by a second bearing (112b) integral to the case (2), which in turn enables the rotation of said second end (111b);

5. An infusion pump for enteral nutrition according to any of the claims 2 to 4 **characterised in that** the pumping mechanism (10) comprises a transmission shaft (13) arranged in parallel to the drive shaft (11); and a transmission belt (14) designed to transmit the rotation of the drive shaft (11) to the transmission shaft (13).

6. An infusion pump for enteral nutrition according to any of the claims 1 to 5 **characterised in that** the pumping mechanism (10) comprises a rotor (15) that has a plurality of perimeter rollers (16) designed to press the feeding tube (5); and a cylindrical chamber (17) into which the rotor (15) is coaxially housed, so the feeding tube (5) is arranged between the cylindrical chamber (17) and the rollers (16).

7. An infusion pump for enteral nutrition according to claim 6 **characterised in that** the cylindrical chamber (17) comprises an inlet opening (171) with an inlet deflector (172), which redirects the feeding tube (5) towards the housing (20), and an output opening (173) with an outlet deflector (174), which redirects the feeding tube (5) towards an output hole (9) of the case (2).

8. An infusion pump for enteral nutrition according to claims 5 and 6 **characterised in that** the drive shaft (11) comprises a toothed perimeter section (113) that meshes with the transmission belt (14); and **in that** the transmission shaft (13) comprises a cogwheel (131) that meshes with the transmission belt (14), and a transmission end (132), integral with the rotor (15).

9. An infusion pump for enteral nutrition according to any of the claims 1 to 8 **characterised in that** the housing (20) comprises a slit (23) which extends downwards from the upper opening (21).

10. An infusion pump for enteral nutrition according to any of the claims 1 to 9 **characterised in that** the case (2) comprises a maintenance opening (24) which provides access to the peristaltic mechanism (10), and which in turn is covered with a detachable plate (25).

11. An infusion pump for enteral nutrition according to claim 10 **characterised in that** the case (2) comprises a closing tab (26) designed to slide over the detachable plate (25) and to enable the attachment thereof on the maintenance opening (24).

12. An infusion pump for enteral nutrition according to any of the claims 1 to 11 **characterised in that** the case (2) comprises a handle (27) designed to be interchangeably assembled either on the first hooking means (28a) arranged on a first side (2a) of the case (2), or on second hooking means (28b) arranged on a second side (2b) of the case (2), opposite to the first side (2a).

13. An infusion pump for enteral nutrition according to any of the claims 1 to 12 **characterised in that** the case (2) comprises a plurality of securing clamps (29) designed to secure an external length (51) of the feeding tube (5).

14. An infusion pump for enteral nutrition according to any of the claims 1 to 13 **characterised in that** it comprises resilient locking members (7) designed to hold the bottle (B) in position once it has been arranged into the housing (20).

15. An infusion pump for enteral nutrition according to any of the claims 1 to 14 **characterised in that** the needle (3) comprises a threaded passage (34) designed to be screwed onto the bottom base (22) of the housing (20).
